# EUROPÄISCHE PATENTSCHRIFT

(11) **EP 2 146 730 B1**
(45) Veröffentlichungstag und Bekanntmachung des Hinweises auf die Patenterteilung: **28.02.2018**
(21) Anmeldenummer: 08733234.2
(22) Anmeldetag: 11.04.2008
(51) Int. Cl.: A61K 36/185, A61P 19/00, A61P 29/00

(54) **PFLANZENEXTRAKT UND SEINE VERWENDUNG**
PLANT EXTRACT, AND USE THEREOF
EXTRAIT VÉGÉTAL ET SON UTILISATION

(30) Priorität: 12.04.2007 AT 5682007
(43) Veröffentlichungstag der Anmeldung: 27.01.2010
(73) Patentinhaber: Steindl, Franz, 1210 Wien (AT); Wiblinger, Michael, 1220 Wien (AT)
(72) Erfinder: STEINDL, Franz, 1210 Wien (AT)
(74) Vertreter: Heger, Georg
(86) Internationale Anmeldenummer: PCT/AT2008/000131
(87) Internationale Veröffentlichungsnummer: WO 2008/124857

(56) Entgegenhaltungen:
- SCHOMAKERS J ET AL: "Urticae herba. Phytochemical and anatomical differentiation of herba drugs from Urtica dioica and Urtica urens" DEUTSCHE APOTHEKER ZEITUNG, DEUTSCHER APOTHEKER VERLAG, STUTTGART, DE, Bd. 135, Nr. 7, 1. Januar 1995 (1995-01-01), Seiten 40-44,46, XP001538748 ISSN: 0011-9857
- DATABASE WPI Week 198935 Thomson Scientific, London, GB; AN 1989-251056 XP002510034 & HU 48 820 A (BALOGH G) 28. Juli 1989 (1989-07-28)
- CHRUBASIK J E ET AL: "A comprehensive review on nettle effect and efficacy profiles, Part I: Herba urticae" PHYTOMEDICINE 20070627 DE, Bd. 14, Nr. 6, 27. Juni 2007 (2007-06-27), Seiten 423-435, XP002510033 ISSN: 0944-7113

## Beschreibung

Die vorliegende Erfindung betrifft ein wässriges Brennnesselextrakt, ein Verfahren zu seiner Herstellung mittels überhitzter wässriger Extraktion sowie die Verwendung des wässrigen Brennnesselextrakts zur Herstellung von Arzneimitteln.

Phytomedizin ist in der Naturheilkunde seit vielen tausend Jahren ein zentraler Teil für die Behandlung vieler Erkrankungen und Leiden. Es wurden auch viele Monosubstanzen der modernen Medizin ursprünglich aus Pflanzen isoliert, z.B. Cytostatika, wie Vicristine (aus Immergrün), Taxol (aus der pazifischen Eibe), oder Schmerzmittel, wie Salicylsäure (aus Weidenrinde), eingesetzt in acetylierter Form als Aspirin, Opiate, etc.

Brennnessel sind seit einigen tausend Jahren als Heilkraut für Mensch und Tiere in Verwendung (http://www.rain-tree.com/nettles.htm und EMEA/MRL/286/97-Final October 1999, Committee for Veterinary Medical products, Urticae Herba, Summary report). Die Anwendungen gegen Schmerzen, Durchblutungsstörungen und Entzündungen erstrecken sich von direkter oberflächlicher Behandlung mit frischen Brennnesseln (Randall, C. et al, "Randomized controlled trial of nettle sting for treatment of base-of-thumb pain." J. R. Soc. Med. 2000; 93(6): 305-9) hin zu oraler Aufnahme von Brennnesseltee und Brennnesselspinat oder Tinkturen, homöopathischen Lösungen oder getrockneten Extrakten, die mittels organischer Lösungsmittel, wie z.B. Äthanol, Isopropanol oder Aceton, gewonnen werden. Im medizinischen Bereich werden Brennnessel und Extrakte daraus seit hunderten von Jahren zur Behandlung von Rheuma, Arthritis, Gicht, Ekzemen und Anämie verwendet.

Die wirksamen Substanzen, die beim Kontakt mit frischen Pflanzen (Härchen an den Blättern und Stängeln) in die Haut injiziert werden, sind Histamin, Serotonin, Choline und Ameisensäure (die für das Brennen hauptverantwortlich ist), vgl. http://www.rain-tree.com/nettles.htm und EMEA/MRL/286/97-Final October 1999, Committee for Veterinary Medical products, Urticae Herba, Summary report.

Als Hauptinhaltsstoffe der Brennnessel sind z.B. die folgenden Verbindungen bekannt: Acetophenole, Acetylcholine, Agglutinine, Alkaloide, Astragalin, Buttersäure, Kaffeesäure, Carbonsäuren, Chlorogensäure, Chlorophyll, Choline, Coumarsäure, Folacin, Ameisensäure, Friedeline, Histamine, Kampherol, Coproporphyrin, Lectine, Lecithin, Lignane, Linolensäure, Neoolivil, Palmatsäure, Pantothensäure, Quercetin, Quinoesäure, Scopoletin, Secoisolariciresinol, Serotonin, Sitosterole, Stigmasterol, Succinsäure, Terpene, Violaxanthin, und Xanthophylle.

In den Wurzeln der Brennnessel sind auch Scopoletin, Sterole, Fettsäuren, Polysaccharide und Isolectine in höheren Konzentrationen vorhanden.

Als pharmakologisch und toxikologisch relevante Komponenten von Brennnesseln werden Flavonoide (Glycoside von Quercitin, Kampferol und Isohamnetin), verschiedene Amine sowie organische und anorganische Säuren eingestuft.

In der heutigen Medizin werden vor allem Extrakte aus Brennnesselwurzeln zur Behandlung von urologischen Problemen während der frühen und fortgeschrittenen Stadien von Prostatavergrößerung (BHP, benign prostatic hyperplasia); Harnwegsinfektionen und bei Nierensteinen verwendet (Safarinejad MR, Urtica dioica for treatment of benign prostatic hyperplasia: a prospective, randomized, double-blind, placebo-controlled, crossover study, J Herb Pharmacother. 2005;5(4):1-11, und Lopatkin N, Sivkov A, Walther C, Schlafke S, Medvedev A, Avdeichuk J, Golubev G, Melnik K, Elenberger N, Engelmann U, Long-term efficacy and safety of a combination of sabal and urtica extract for lower urinary tract symptoms - a placebo-controlled, double-blind, multicenter trial, World J Urol., 2005 Jun; 23(2):139-46, Epub 2005 Jun 1, und http://www.metagenics.com/resources/imc- /onemedicinecons/ConsHerbs/StingingNettlech.html).

Die US 2005/0175717 A1 offenbart eine Perkolationsmethode für Pflanzenbestandteile, darunter auch der Brennnessel, mittels eines Perkolationsfluids, welches zumindest Dampf umfasst, welches Verfahren z.B. in einer handelsüblichen Espressomaschine bei einer Temperatur von 40°C bis 120°C und einem Druck von mindestens 3 bar durchgeführt wird. Das so gewonnene Extrakt wird zur kosmetischen Behandlung von Keratin verwendet. Der Vorteil dieses Verfahrens liegt darin, dass derartige Extrakte innerhalb kürzester Zeit, beispielsweise in weniger als 2 Minuten, herstellbar sind.

Gemäß der HU 40009 A2, Lutomski J. et al, "Die Brennessel in Heilkunde und Ernährung", Pharmazie in unserer Zeit, 12. Jahrg., 1983, Nr. 6, Seiten 181-186 und WO 1993/013754 A1 werden Dekokte durch Kochen der Pflanzenbestandteile in Wasser gewonnen.

Aufgabe der vorliegenden Erfindung ist es, ein gegenüber dem Stand der Technik wirksameres wässriges Brennnesselextrakt vorzusehen.

Laut Literatur ist das Molekül 2-O-Caffeoylmalat der Hauptwirkstoff für die Schmerzlinderung in Extrakten aus Brennesseln (Cyclo-oxygenase- und 5-Lipo-oxigenase-Hemmer) (Obertreis B, Giller K, Teucher T, Behnke B, Schmitz H, Antiinflammatory effect of Urtica dioica folia extract in comparison to caffeic malic acid, Arzneimittelforschung, 1996 Jan, 46(1):52-6). Es gibt allerdings auch Studien, die bei oraler Aufnahme keinen direkten Zusammenhang zwischen der Konzentration von 2-O-Caffeoylmalat und der Wirkung festgestellt haben. Im Zuge der Arbeiten zur vorliegenden Erfindung wurde festgestellt, dass es mehrere Substanzen sind, welche schmerzlindernd wirken, von denen die Meisten jedoch durch HPLC-Analysen nicht zu trennen sind.

Im Zuge der Arbeiten zur vorliegenden Erfindung wurde ein wässriges Brennnesselextrakt hergestellt, welches unter anderen Peaks (A) bei 23,66 ± 0,1 min, (B) bei 40,79 ± 0,1 min und (C) bei 44,89 min ± 0,1 min Retentionszeit umfasste, wobei Peak (A) eine Peakfläche von mindestens 50 mAU*s und Peaks (B) und (C) jeweils eine Peakfläche von mindestens 100 mAU*s aufweisen, gemessen mittels HPLC (HP-1100 der Fa. Agilent, Waldbronn, Deutschland, C18-Säule vom Typ HP 1100-32, 150 x 4,6 mm, Partikelgröße 5µm, Laufmittel 0-70 Vol.-% Methanol + 1 Vol.-% Eisessig zu destilliertem Wasser im Gradientenverfahren in 100 min) bei einem Injektionsvolumen von 20 µl und Detektion bei 328 nm. In weiterer Folge ist es gelungen die Meisten der für die obgenannten Peaks im wässrigen Brennnesselextrakt verantwortlichen Wirkstoffe zu identifizieren, es handelt sich bei den für Peak (B) bei 40,79 ± 0,1 min verantwortlichen Substanzen um ein Gemisch von p-Coumarinsäure und einem Caffeoylmalat, bei Peak (C) bei 44,89 min ± 0,1 min um ein Gemisch von Coumarin und trans-Ferulasäure.

Der im erfindungsgemäßen wässrigen Brennnesselextrakt unter anderen auch enthaltene Peak (D) bei 34,12 ± 0,1 min Retentionszeit, gemessen mittels HPLC (HP-1100 der Fa. Agilent, Waldbronn, Deutschland, C18-Säule vom Typ HP 1100-32, 150 x 4,6 mm, Partikelgröße 5µm, Laufmittel 0-70 Vol.-% Methanol + 1 Vol.-% Eisessig zu destilliertem Wasser im Gradientenverfahren in 100 min) bei einem Injektionsvolumen von 20 µl und Detektion bei 328 nm, wurde als Kaffeesäure identifiziert..

Diese Identifizierungen stimmen auch mit der präparativen Isolierung des erfindungsgemäßen wässrigen Brennnesselextrakts in der LC-MS-Analyse zusammen, zeigte diese doch ein ganzes Spektrum von potentiellen Cyclo-oxygenase- und Lipo-oxigenase-Hemmern, nämlich neben dem 2-O-caffeoylmalat auch die trans-Ferulasäure und auch mehrfach Kombinationen aus Kaffeesäure und Apfelsäure, wie von den Inhaltsstoffen her schon durch Nielsen JK et.al (Nielsen JK, Olsen O, Pedersen LH, Sørensen H (1984) 2-O-(p-coumaroyl)-L-malate, 2-O-caffeoyl-L-malate and 2-O-feruloyl-L-malate in Raphanus Sativus. Phytochemistry 23: 1741-1743) gefunden wurde. Kaffeesäure und Ferulasäure gehören zu den am häufigsten vorkommenden sekundären Pflanzenstoffen in Nahrung auf pflanzlicher Basis.

Gegenstand der vorliegenden Erfindung ist somit ein wässriges Brennnesselextrakt umfassend ein Gemisch von p-Coumarinsäure und einem Caffeoylmalat sowie ein Gemisch von Coumarin und trans-Ferulasäure, und zwar mit einer Mindestkonzentration von 0,75 µg/ml an Gemisch von p-Coumarinsäure und einem Caffeoylmalat und einer Mindestkonzentration von 0,80 µg/ml an Gemisch von Coumarin und trans-Ferulasäure, bei Extraktion von 4 g getrocknete und zerkleinerte Blätter und Stängel mit 96 ml RO oder HQ-Wasser. Durch Vergleichsversuche wurde gefunden, dass wässrige Brennnesselextrakte hergestellt mittels eines Verfahrens des Standes der Technik, d.h. mit einer Extraktion unterhalb 120°C, einen Gehalt von maximal 0,66 µg/ml des Gemisches von p-Coumarinsäure und einem Caffeoylmalat bzw. maximal 0,48 µg/ml des Gemisches von Coumarin und trans-Ferulasäure enthalten.

Weiters wird bevorzugt, wenn das erfindungsgemäße wässrige Brennnesselextrakt auch Kaffeesäure umfasst, und zwar in einer Mindestkonzentration von 1,50 µg/ml bei Extraktion von 4 g getrocknete und zerkleinerte Blätter und Stängel mit 96 ml RO oder HQ-Wasser.

Vorzugsweise umfasst das erfindungsgemäße wässrige Brennnesselextrakt ein Gemisch von p-Coumarinsäure und einem Caffeoylmalat, ein Gemisch von Coumarin und trans-Ferulasäure sowie Kaffeesäure, und zwar mit einer Mindestkonzentration von 0,90 µg/ml an Gemisch von p-Coumarinsäure und einem Caffeoylmalat, einer Mindestkonzentration von 1,05 µg/ml an Gemisch von Coumarin und trans-Ferulasäure sowie einer Mindestkonzentration von 2,00 µg/ml Kaffeesäure.

Besonders bevorzugt umfasst das erfindungsgemäße wässrige Brennnesselextrakt ein Gemisch von p-Coumarinsäure und einem Caffeoylmalat, ein Gemisch von Coumarin und trans-Ferulasäure sowie Kaffeesäure, und zwar mit einer Mindestkonzentration von 1,00 µg/ml an Gemisch von p-Coumarinsäure und einem Caffeoylmalat, einer Mindestkonzentration von 1,10 µg/ml an Gemisch von Coumarin und trans-Ferulasäure sowie einer Mindestkonzentration von 2,70 µg/ml Kaffeesäure.

In einem weiteren Aspekt wird durch die vorliegende Erfindung ein Verfahren zur Herstellung des erfindungsgemäßen wässrigen Brennnesselextrakts zur Verfügung gestellt, wobei Brennnesseln (Urtica dioica/Urtica urens, Stinging Nettle) mittels Wasser bei einer Temperatur von 120 bis 140°C, unter Druck extrahiert werden. Es hat sich gezeigt, dass ein Verfahren mittels überhitzter wässriger Extraktion von Brennnesseln (Urtica dioica/Urtica urens, Stinging Nettle) zu einer völlig anderen Konzentration und Zusammensetzung der Inhaltsstoffe und einer wesentlich besseren und konstanteren Wirksamkeit dieses Extraktes im Vergleich zu anderen Extraktionsmethoden führt. Die so gewonnenen Extrakte zeigen eine wesentlich höhere anti-Schmerz-, durchblutungsfördernde und entzündungshemmende Wirkung, als mit bekannten Extraktionsmethoden erreicht werden kann. Darüber hinaus ist von Vorteil, dass im erfindungsgemäßen Verfahren keine organischen Lösungsmittel im Prozess verwendet werden müssen. Ein weiterer Vorteil dieser Extraktionsmethode ist, dass die in der Brennnessel vorhandene Ameisensäure einen Siedepunkt von über 100°C aufweist, jedoch bei Überschreiten ihres Siedepunktes in Anwesenheit von Sauerstoff in CO₂ und H₂O zerfällt. Durch das erfindungsgemäße Verfahren mit einer wässrigen Extraktion bei einer Temperatur von 120 bis 140°C wird somit die Ameisensäure im Extrakt stark abgereichert. Das erfindungsgemäße Extraktionsverfahren ist nicht darauf ausgelegt, das Maximum an begrenzt wasserlöslichen Wirksubstanzen zu extrahieren (wie es z.B. mit häufig verwendeten organischen Lösungsmitteln, Isopropanol, Aceton, etc., möglich ist), sondern um einen konstanten Wirkstoffgehalt zu erhalten. Hierzu wird das erhaltene Extrakt im Abkühlungsprozess bei einer Temperatur von mindestens etwa 80 bis 90°C, vorzugsweise mindestens 85°C filtriert. Wenn möglich werden die Extrakte sofort warm weiterverarbeitet.

Gemäß einer bevorzugten Ausführungsform des erfindungsgemäßen Verfahrens wird zur Extraktion Wasser in einer Menge von 85 bis 99 %-Masse, vorzugsweise 90 bis 97 %-Masse, besonders bevorzugt 96 %-Masse, bezogen auf die Gesamtmasse der zu extrahierenden Zusammensetzung, eingesetzt.

Besonders bevorzugt wird die Extraktion über einen Zeitraum von 1 bis 60 min durchgeführt, d.h. die zu extrahierende Zusammensetzung wird über einen Zeitraum von 1 bis 60 min auf der gewählten Extraktionstemperatur gehalten. Die Zeitdauer der Extraktion wirkt sich auf die Abreicherung von flüchtigen Substanzen aus. Bevorzugt werden 20-30 min Extraktionsdauer.

Weiters wird bevorzugt, dass im erfindungsgemäßen Verfahren 1 bis 10 %-Masse, vorzugsweise 5 bis 7 %-Masse, besonders bevorzugt 4 %-Masse, Brennnesseln (Urtica dioica/Urtica urens, Stinging Nettle, vorzugsweise in Form getrockneter und zerkleinerter Blätter und Stängel, bezogen auf die Gesamtmasse der zu extrahierenden Zusammensetzung, eingesetzt werden. Die zu extrahierende Zusammensetzung liegt somit vorzugsweise in Form einer 1 bis 10%igen, vorzugsweise in Form einer 5 bis 7%igen, besonders bevorzugt als 4%ige Lösung vor.

Der erhaltene Extrakt wird in höheren Konzentrationen in Form von Cremen verarbeitet und lokal (topisch) appliziert, oder auch oral als reiner Extrakt.

Ein weiterer Aspekt der vorliegenden Erfindung betrifft somit die Verwendung des erfindungsgemäßen wässrigen Brennnesselextrakts zur Herstellung einer pharmazeutischen Zubereitung, insbesondere zur Behandlung von Entzündungen, Durchblutungsstörungen und/oder Schmerzen. Hierzu wird der Extrakt in hitzestabilen Glasgefäßen in z.B. einem geeigneten Autoklaven erhitzt, nach dem Abkühlen auf 95-100°C entnommen, bei mindestens 85°C durch ein Papierfilter filtriert und bei über 80°C der Creme-Grundbasis in entsprechender Menge (etwa 10-75 %-Masse) beigemengt. Die lokale Applikation als Creme ermöglicht dabei eine Reduktion von Wirkstoffen um einige Zehnerpotenzen im Vergleich zur oralen Applikation. Falls die filtrierten Extrakte vor ihrer Weiterverarbeitung zwischengelagert werden, können möglicherweise aufgrund der bei niedrigeren Temperaturen zu geringen Löslichkeit ausgefallene Substanzen in geeigneten Lösungsmitteln (z.B. der zur Neutralisierung der Creme verwendeten Lauge) wieder quantitativ gelöst und der Creme so zugefügt werden.

Die folgenden Beispiele und Figuren dienen der Erläuterung der vorliegenden Erfindung, ohne sie auf die jeweiligen Ausführungsformen zu beschränken.

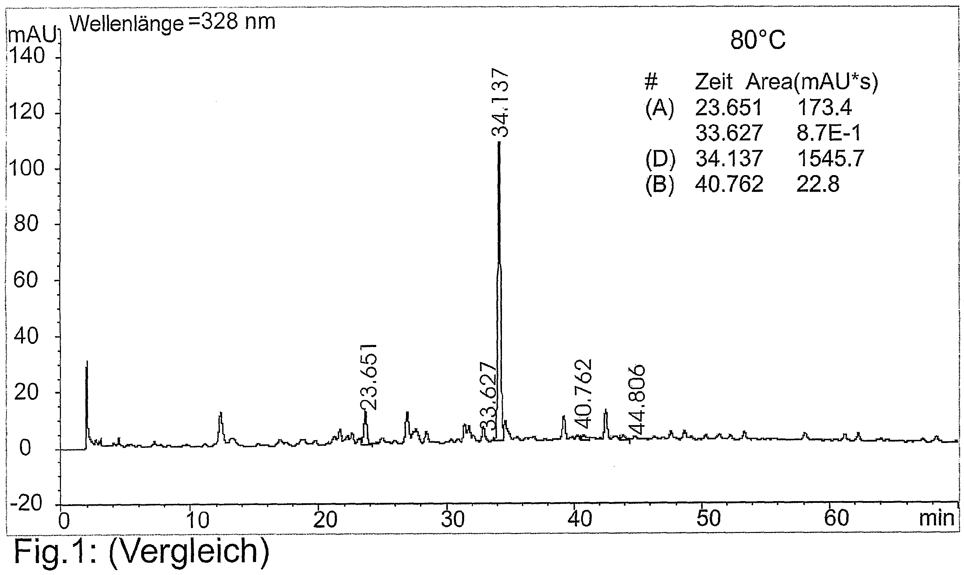

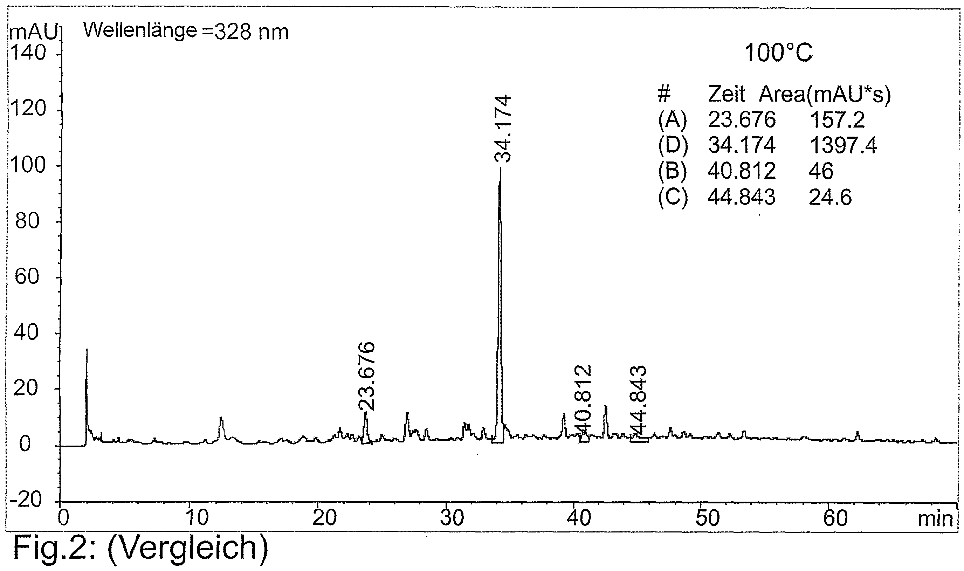

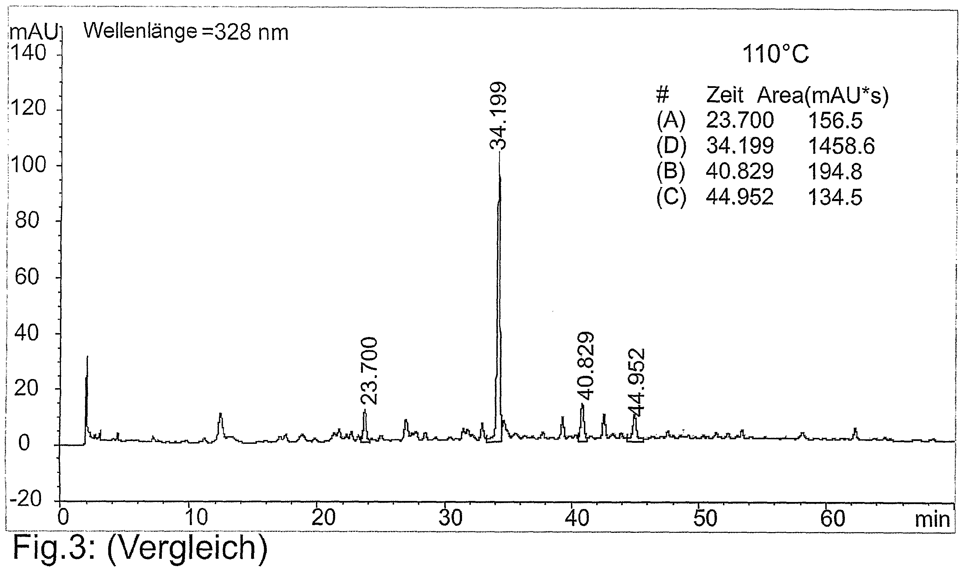

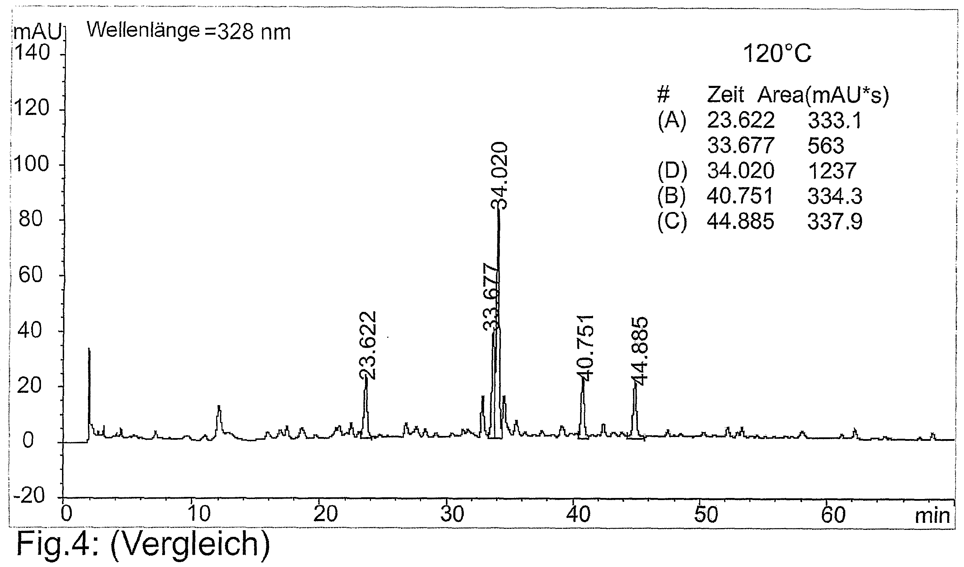

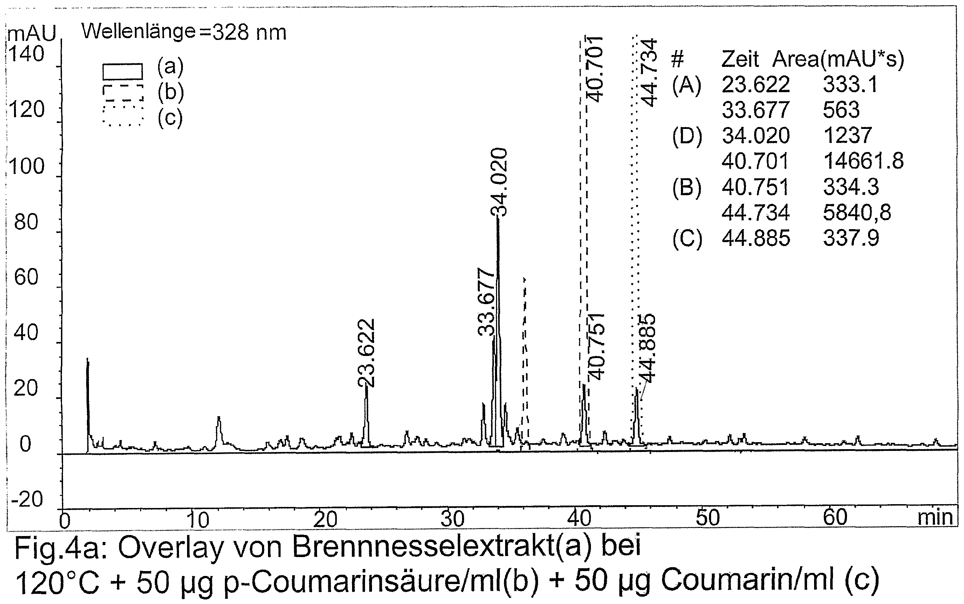

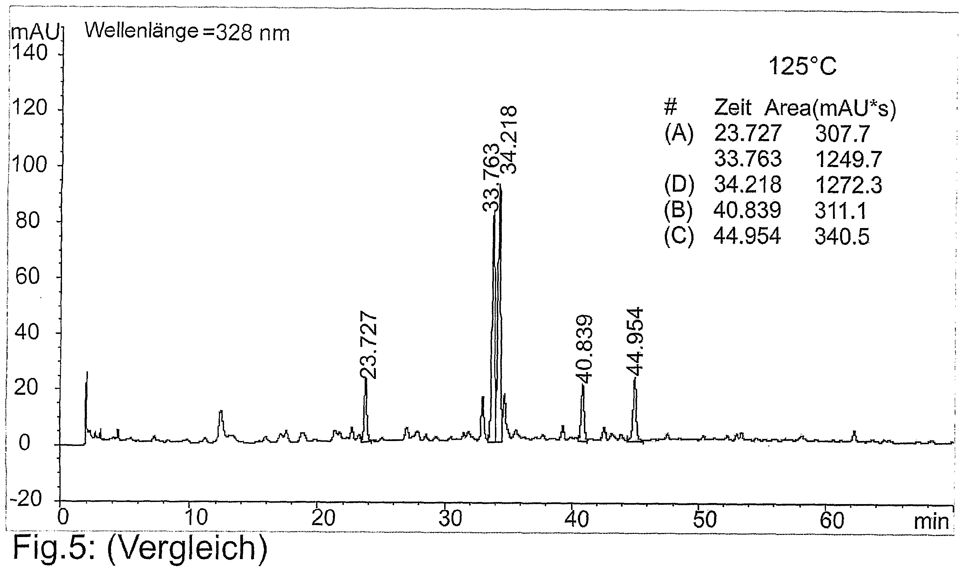

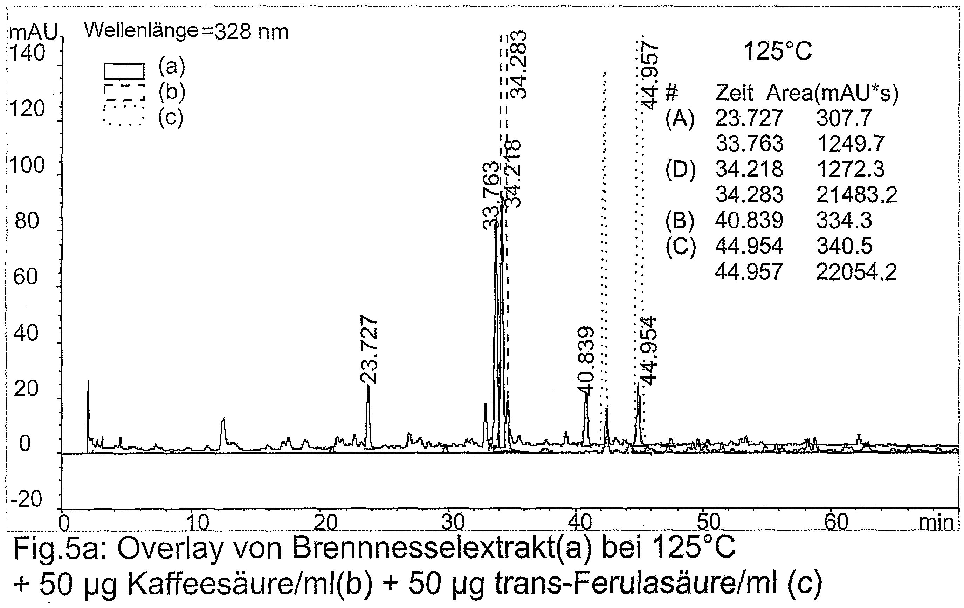

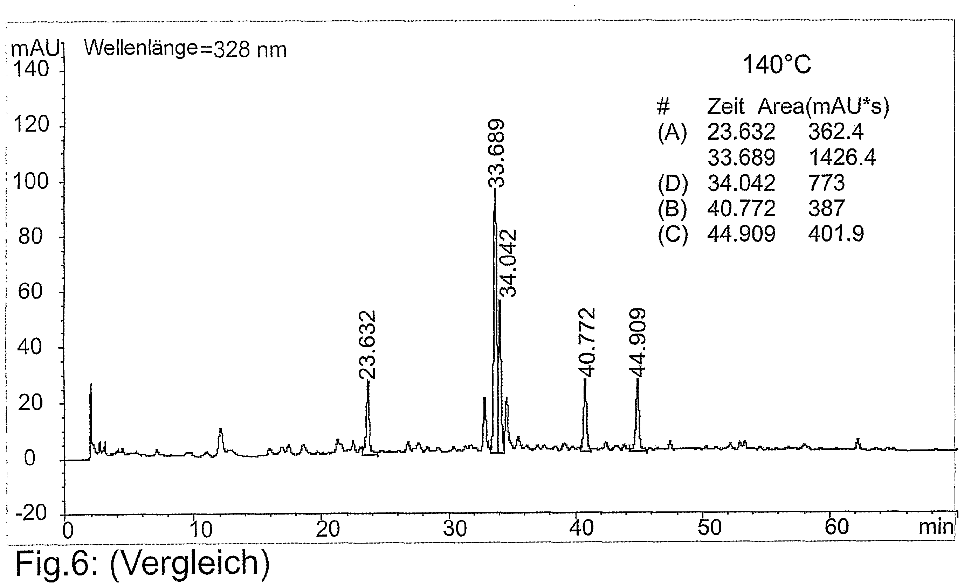

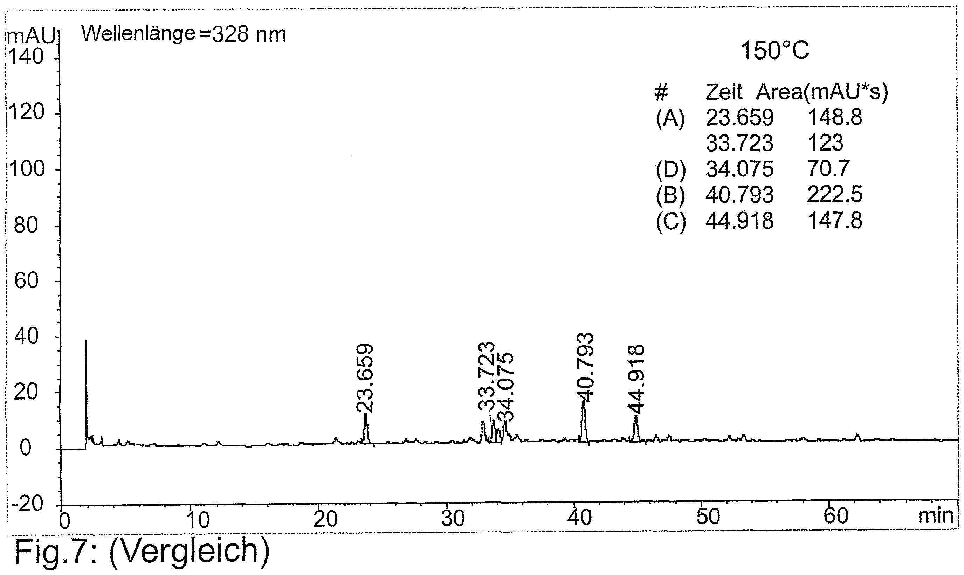

In den Figuren 1 bis 7 werden HPLC-Analysen an Extrakten gezeigt, welche mittels verschiedener Extraktionstemperaturen erhalten wurden. Die zu extrahierenden Zusammensetzungen wurden jeweils 30 min lang auf folgender Temperatur gehalten: 80°C (Fig. 1, Vergleich), 100°C (Fig. 2, Vergleich), 110°C (Fig. 3), 120°C (Fig. 4), 125°C (Fig. 5), 140°C (Fig. 6), und 150°C (Fig. 7). Die HPLC-Analysen wurden bei Raumtemperatur mittels einer Apparatur vom Typ HP-1100 (Fa. Agilent, Waldbronn, Deutschland) durchgeführt. Als Trennsäule wurde eine C18-Säule vom Typ HP 1100-32 verwendet, in der Dimension 150 x 4,6 mm, Partikelgröße 5µm. Als Laufmittel wurde im Gradientenverfahren 0-70 Vol.-% Methanol + 1 Vol.-% Eisessig zu destilliertem Wasser in 100 min benutzt. Nach 70 min Laufzeit sind keine Änderungen oder signifikanten Peaks in Extrakten von 40-170°C mehr erkennbar und werden daher auch in den Abbildungen Fig. 1 - Fig. 7 nicht gezeigt.

Als minimales Qualitätskriterium wurde festgelegt, dass die Peakfläche der 3 Peaks mit den Elutionszeiten von (A) 23,66 ± 0,1 min, (B) 40,79 ± 0,1 min und (C) 44,89 min ± 0,1 min, detektiert bei 328 nm, für Peak (A) mindestens 50 mAU*s und für Peaks (B) und (C) jeweils mindestens 100 mAU*s betragen muss. Wie klar ersichtlich ist, wird der Peak bei 34,12 ± 0,1 min Laufzeit (enthält Kaffeesäure) über 110°C in mehrere Peaks aufgespalten, das heißt es entstehen Produkte, die unterschiedlich polar sind, bzw. werden andere Inhaltsstoffe bei dieser Temperatur erst löslich.

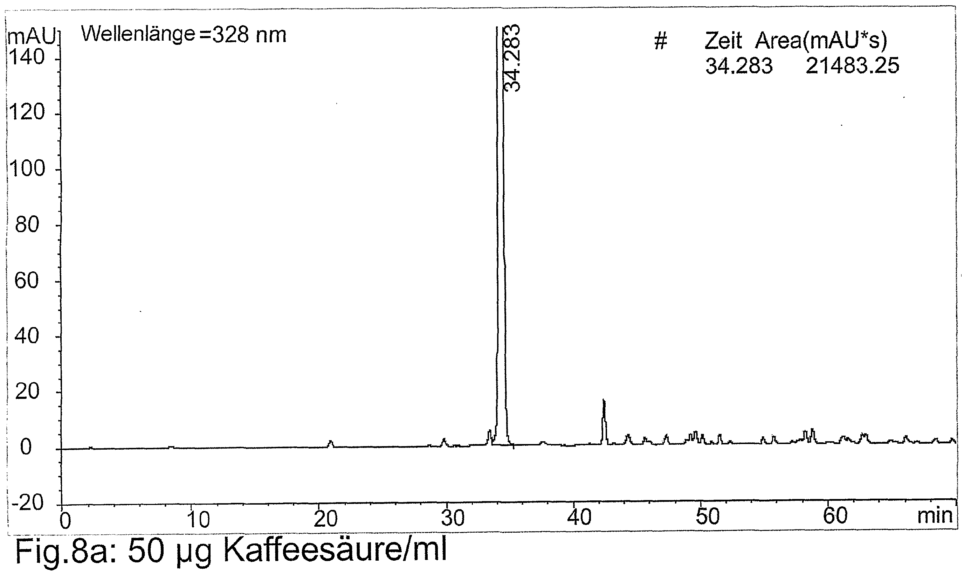

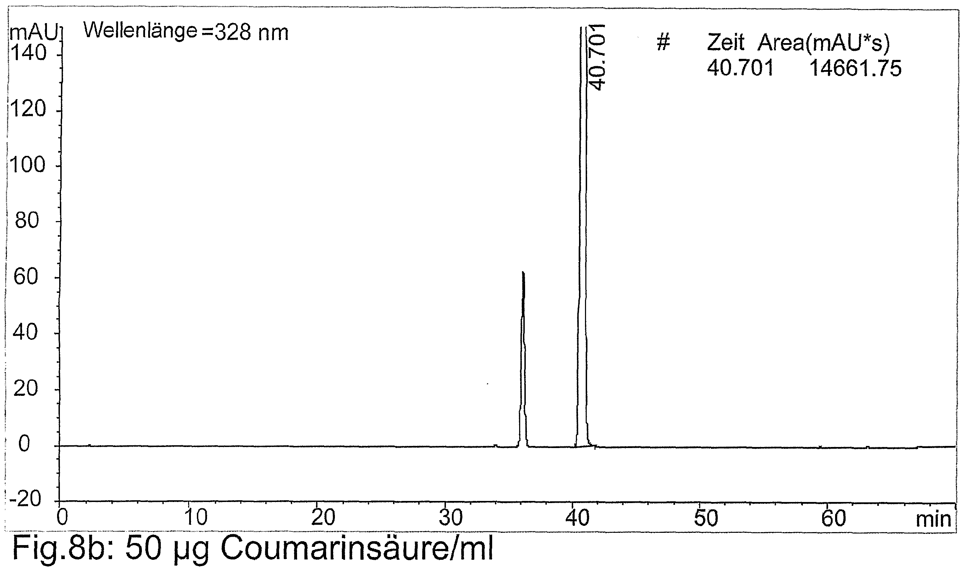

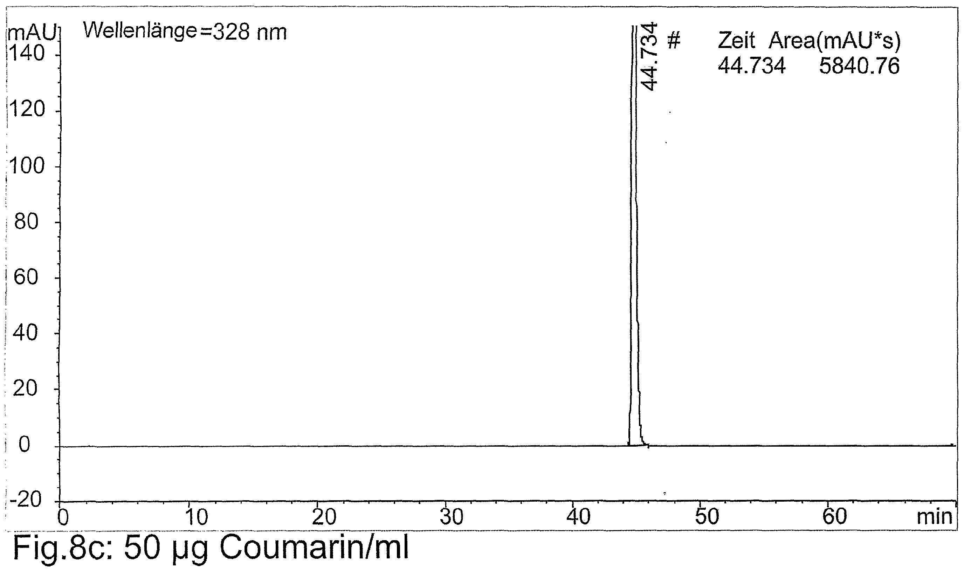

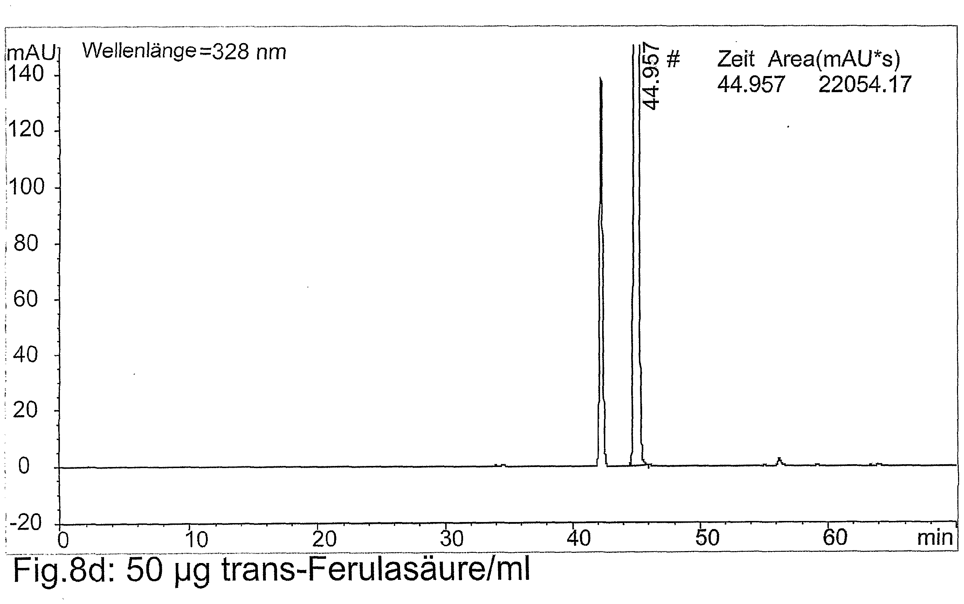

Wie bereits ausgeführt konnten einige Peaks im erfindungsgemäßen Brennnesselextrakt identifiziert werden, und zwar durch Zugabe von Spikes von Reinstsubstanzen in parallelen Proben. In den Fig. 8a bis d sind HPLC-Analysen (Verfahren und Vorrichtung analog zu den in Fig. 1 bis 7 verwendeten) der entsprechenden Reinsubstanzen gezeigt, in Fig. 8a von Kaffeesäure mit einer Konzentration von 50 µg/ml, in Fig. 8b von p-Coumarinsäure mit einer Konzentration von 50 µg/ml, in Fig. 8c von Coumarin mit einer Konzentration von 50 µg/ml und in Fig. 8d von trans-Ferulasäure mit einer Konzentration von 50 µg/ml. In den Fig. 4a und 5a wurden, ausgehend von den obgenannten Reinsubstanzen, die Peaks der HPLC-Analysen identifiziert.

Beispiele:

### Beispiel 1: Herstellung eines Brennnesselextraktes

4 g getrocknete und zerkleinerte Blätter und Stängel von Brennnesseln (Urtica dioica/Urtica urens, Stinging Nettle) werden in eine hitzestabile Glasflasche mit Schraubverschluss (200ml oder 500ml von z.B. Fa. Schott) transferiert und 96 ml RO- oder HQ-Wasser werden zugegeben. Der Inhalt wird in der zugeschraubten Flasche gemischt, der Schraubverschluss wird mindestens eine Umdrehung geöffnet und die Flasche wird in einen geeigneten Autoklaven gestellt. Nach dem Erreichen von 140°C wird die Temperatur 30 min beibehalten und dann lässt man vor dem Öffnen des Autoklaven auf 95-98°C abkühlen. Der Inhalt der Flasche wird so rasch wie möglich, jedenfalls bei mindestens 85°C, durch ein Papierfilter filtriert. Bei großen Mengen (Gefäßen) kann die Restmenge zwischendurch in einem vorgeheizten Wasserbad (90-95°C) warm gehalten werden. Das filtrierte Extrakt wird entweder sofort weiterverarbeitet (z.B. zu einer Creme) oder nochmals in einem Wasserbad auf 90°C erwärmt und verschlossen aufbewahrt.

### Beispiel 2: Herstellung einer 60 %igen Creme

Das in Beispiel 1 erhaltene Extrakt wird in einem Wasserbad auf 80°C temperiert. Dann wird das Extrakt in ein Produktionsgefäß mit starkem Rührwerk transferiert und auf 90°C erwärmt, Glycerinmonostearat wird unter starkem Rühren portionsweise zugegeben und innerhalb von 15 min gelöst. Die Zugabe der weiteren Zutaten It. Tabelle 1 kann schneller erfolgen, mit Ausnahme des Carbomer 940, dieses lässt man am besten einen Tag in den 3 Liter RO Wasser vorquellen und transferiert es in kleineren Portionen, um Klumpenbildung zu vermeiden.

**Tabelle 1**

| Einwaage für 30kg Creme | Temp. (im Rührgefäß) | Charge LOT3460 211106 30kg |
|---|---|---|
| Brennnesselextrakt, hergestellt wie in Bsp. 1 | 90°C | 60 % = 18 Liter |
| Glycerinmonostearat | 90°C | 5 % = 1500 g |
| Olivenöl, Distelöl, Ricinusöl, Mandelöl 1:1:1:1 | 80°C | 5 % = 1500 Milliliter je 375 ml |
| Carbomer 940 | 75°C | 0,85 % = 255 g |
| AloeVera Gel | 75°C | 2,5 % = 750 ml |
| Lanolin | 75°C | 0,5 % = 150 g |
| Tego Betain | 75°C | 3 % = 900 ml |
| Allantoin | 75°C | 1 % = 300 g |
| Paraffinöl | 75°C | 1 % = 300 g |
| Triethanolamin | 65°C | 30 ml (-) |
| Germaben II E | 50°C | 1 % = 300 ml |
| Geruchsessenz z.B. Pfirsich | 50°C | 0,02 % = 6 ml |
| pH einstellen mit 1M Lauge | 50°C | auf pH 6,6 - 6,8 |
| RO Wasser + Carbomer 940 | 50°C | 3000 ml |
| Rühren bis 25°C dann Kühlstellen | | |

Zur Qualitätssicherung und zur Bestimmung der aus dem Extrakt stammenden Inhaltsstoffe der Creme wird eine bestimmte Menge (hier nachfolgend 100 g) eingewogen und unter ständigem Rühren mit 1 N HCl auf pH 2,5 bis 3 eingestellt. Die entmischte Emulsion wird anschließend bei 20°C 15 min bei mindestens 15.000g zentrifugiert.

Die aus der Creme (Cremebasis) stammende, aufgeschwommene und verhärtete Fettschicht (Glycerinmonostearat) wird mit einem spitzen Gegenstand durchstochen (z.B. mit einer sauberen Pipettenspitze) und die darunter liegende flüssige Phase wird abgesaugt und über ein Papierfilter filtriert. Das Filtrat wird mit 1 N Lauge wieder auf pH 6,6 bis 6,8 eingestellt.

Das auf pH 6,6 bis 6,8 eingestellte Filtrat wird nochmals 15 min bei mindestens 15.000g zentrifugiert und dann mittels HPLC, wie oben für Fig. 1 bis 8 beschrieben, analysiert. Die Ergebnisse der Untersuchungen sind in den Figs. 9 und 10 gezeigt.

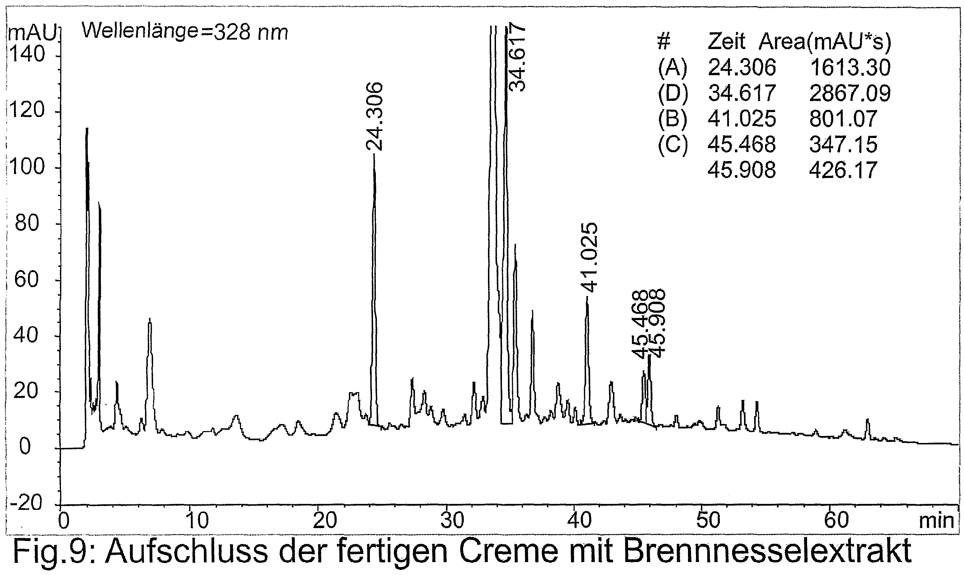

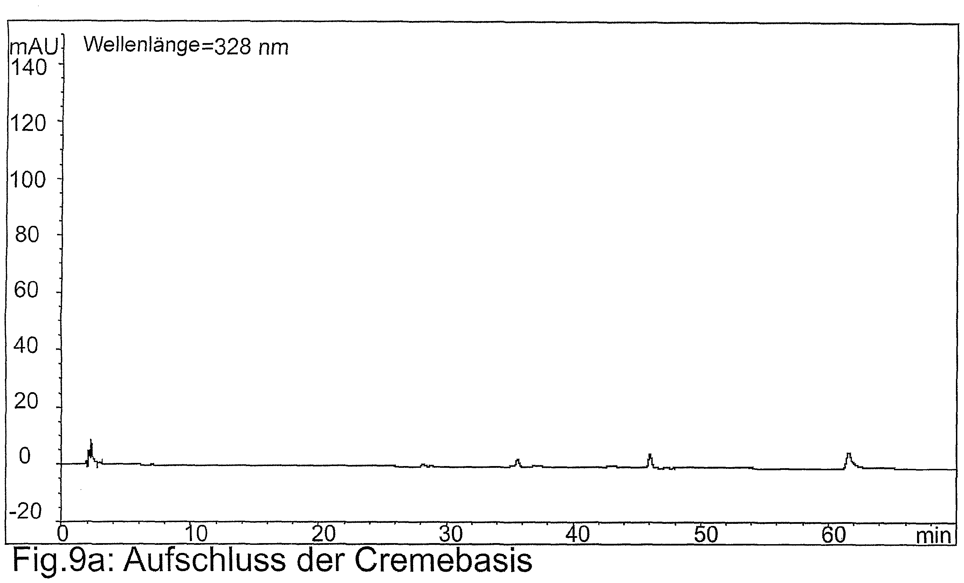

In Fig 9 ist eine HPLC-Analyse der Creme enthaltend das erfindungsgemäße Extrakt dargestellt, besser gesagt die HPLC-Analyse der wie oben erwähnt aufgeschlossenen fertigen Creme mit dem erfindungsgemäßen Brennnesselextrakt.

Fig. 9a stellt die HPLC-Analyse der wie oben erwähnt aufgeschlossenen Cremebasis (d.h. ohne dem erfindungsgemäßen Brennnesselextrakt) dar und dient als Vergleich.

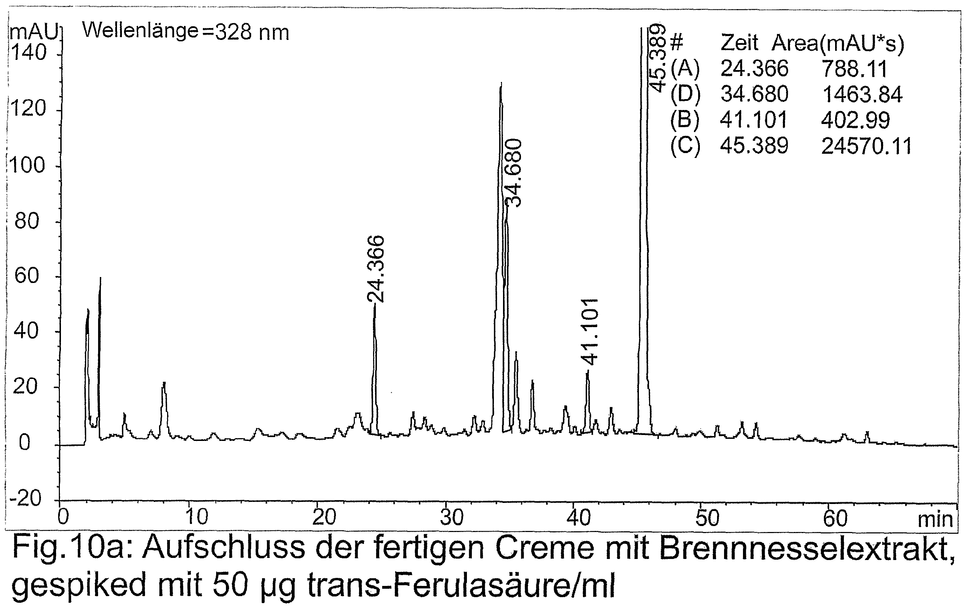

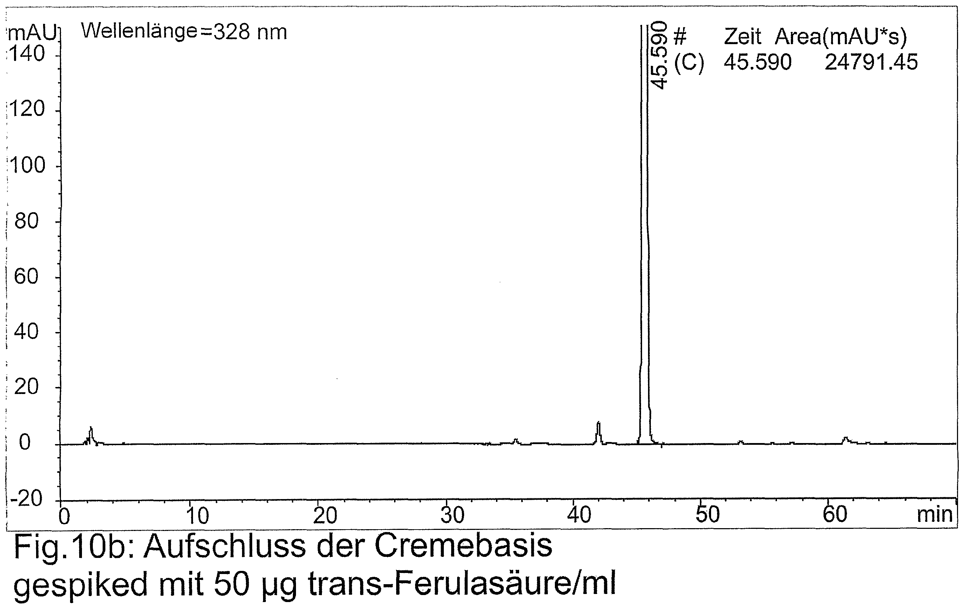

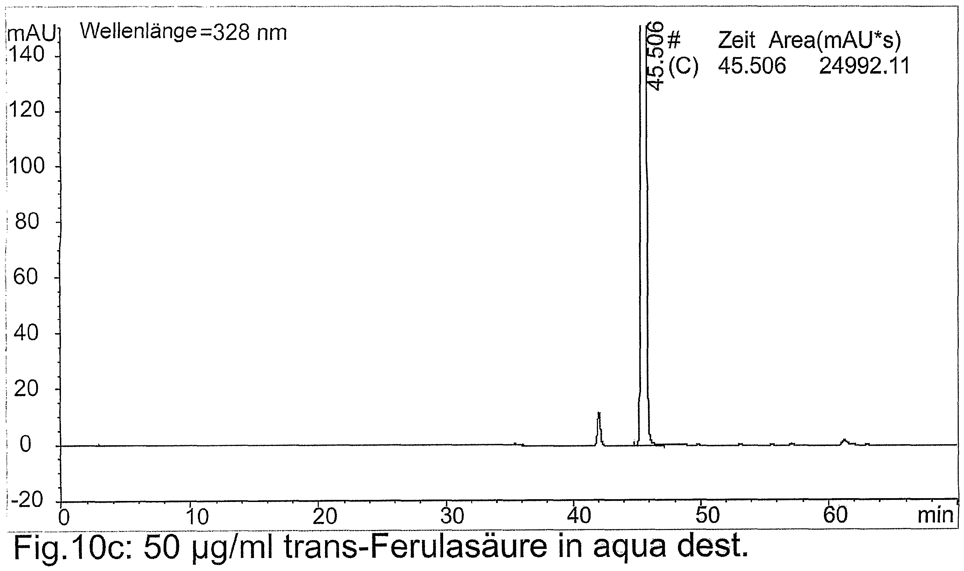

Figs 10a bis c zeigen eine HPLC-Analyse der aufgeschlossenen Creme enthaltend das erfindungsgemäße Extrakt, gespiked mit 50 µg/ml trans-Ferulasäure (Fig 10a), eine HPLC-Analyse der aufgeschlossenen Cremebasis gespiked mit 50 µg/ml trans-Ferulasäure (Fig 10b) und eine HPLC-Analyse der Reinsubstanz 50 µg/ml trans-Ferulasäure (Fig 10c) in Aqua dest.

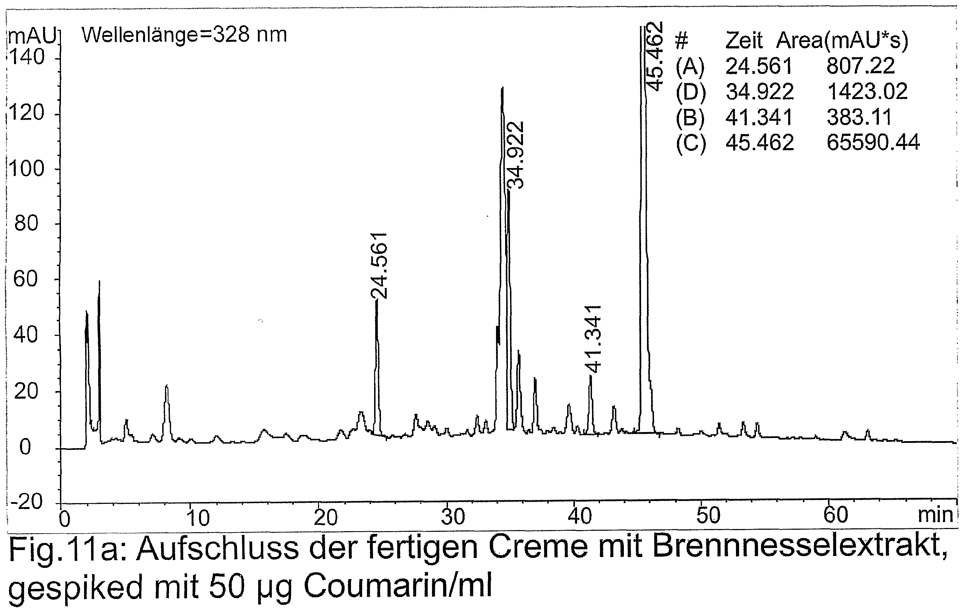

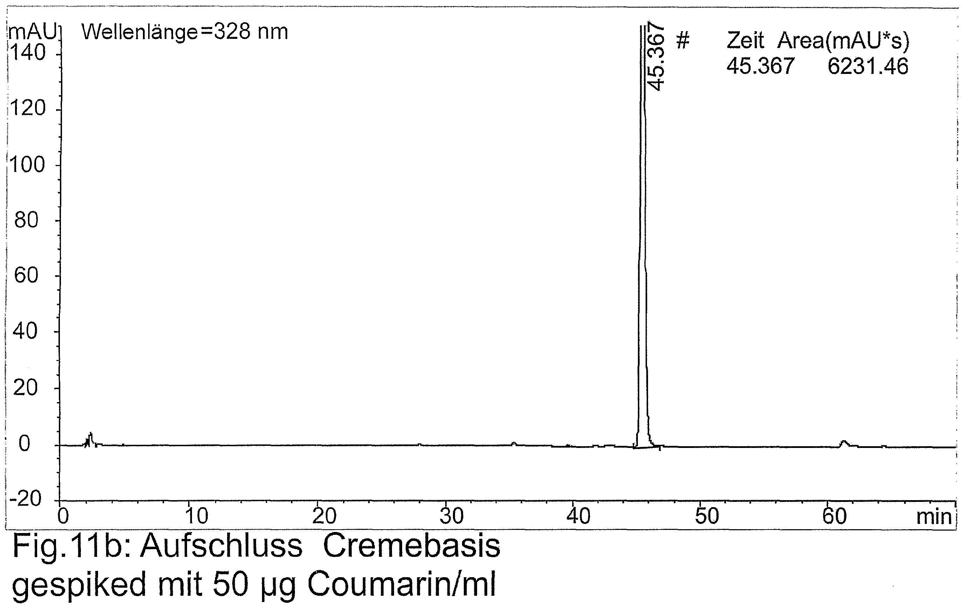

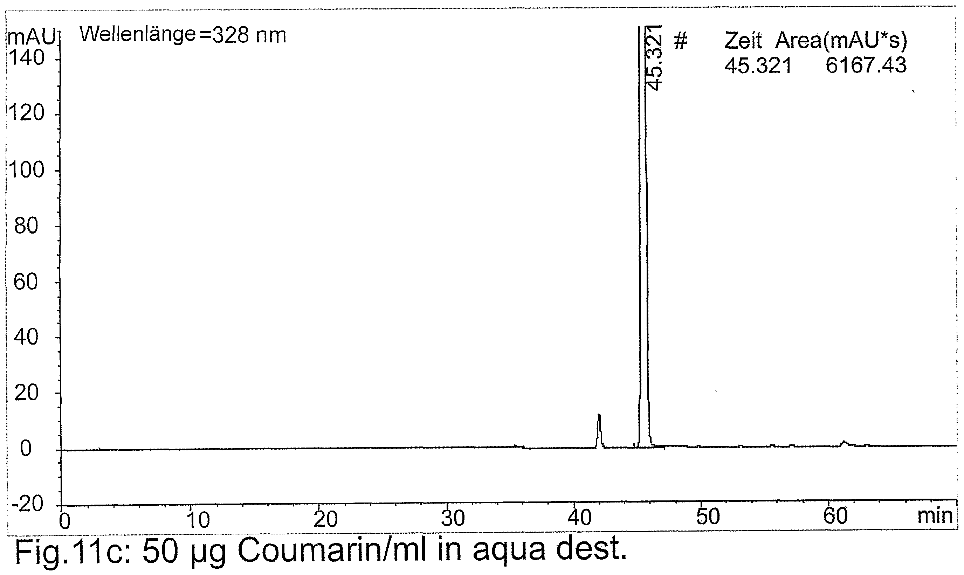

Figs 11a bis c zeigen in gleicher Weise eine HPLC-Analyse der aufgeschlossenen Creme enthaltend das erfindungsgemäße Extrakt, gespiked mit 50 µg/ml Coumarin (Fig 11a), eine HPLC-Analyse der aufgeschlossenen Cremebasis gespiked mit 50 µg/ml Coumarin (Fig 11b) und eine HPLC-Analyse der Reinsubstanz 50 µg/ml Coumarin (Fig 11c) in Aqua dest.

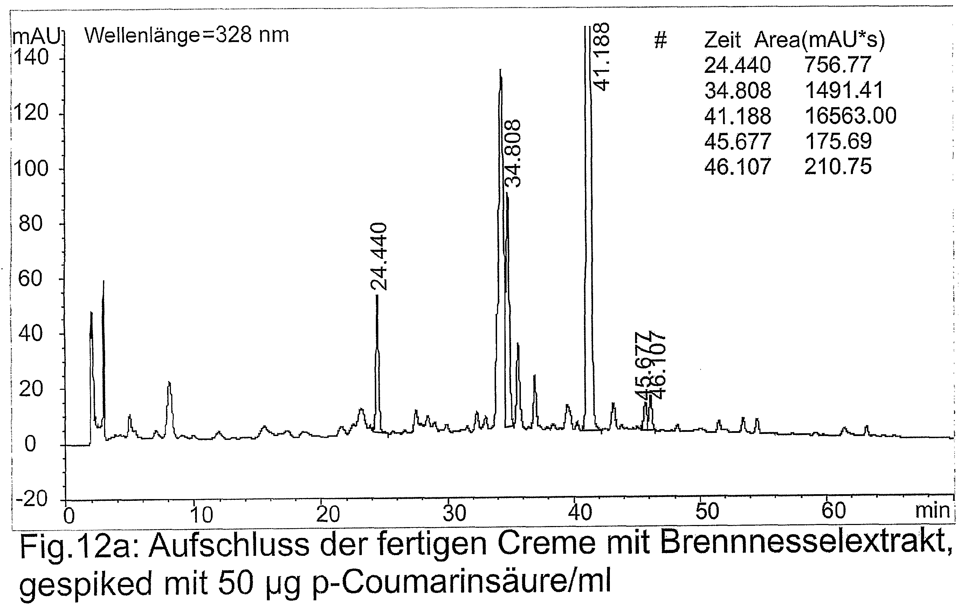

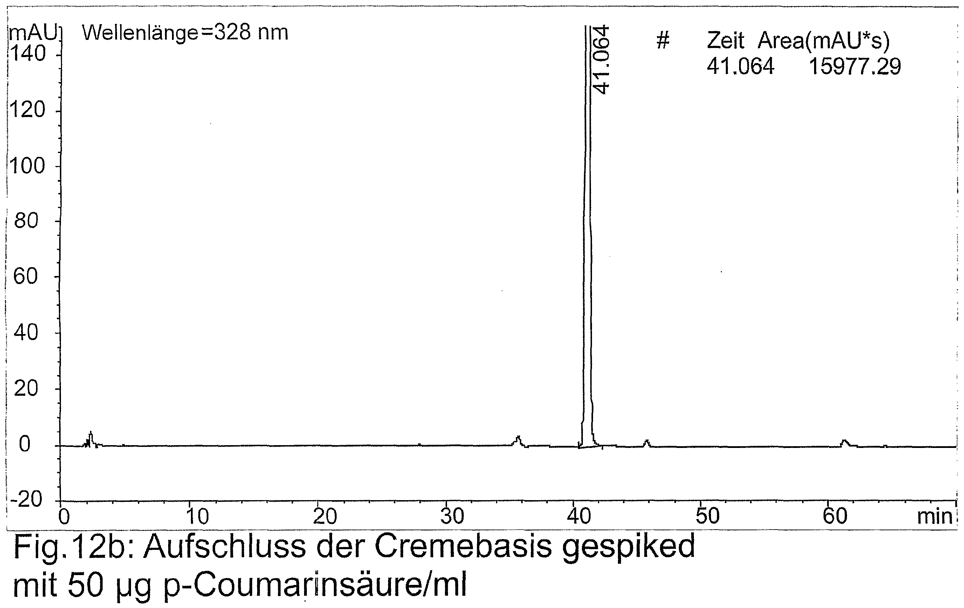

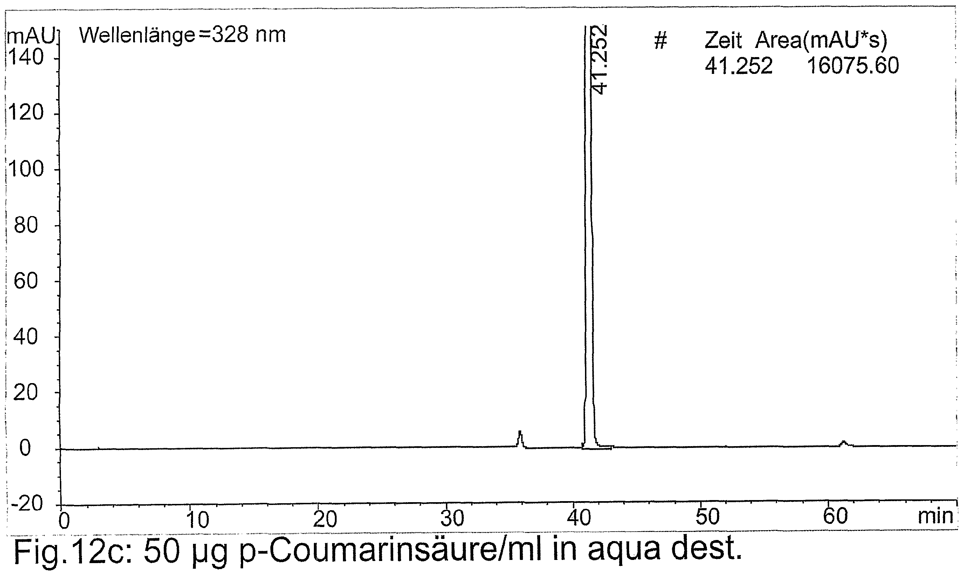

Figs 12a bis c zeigen eine HPLC-Analyse der aufgeschlossenen Creme enthaltend das erfindungsgemäße Extrakt, gespiked mit 50 µg/ml p-Coumarinsäure (Fig 12a), eine HPLC-Analyse der aufgeschlossenen Cremebasis gespiked mit 50 µg/ml p-Coumarinsäure (Fig 12b) und eine HPLC-Analyse der Reinsubstanz 50 µg/ml p-Coumarinsäure (Fig 12c) in Aqua dest.

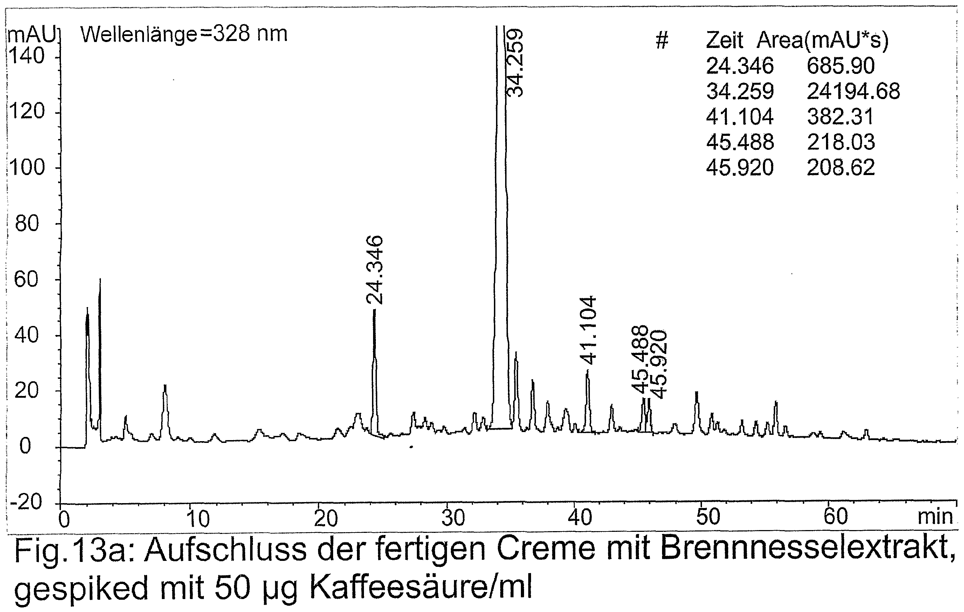

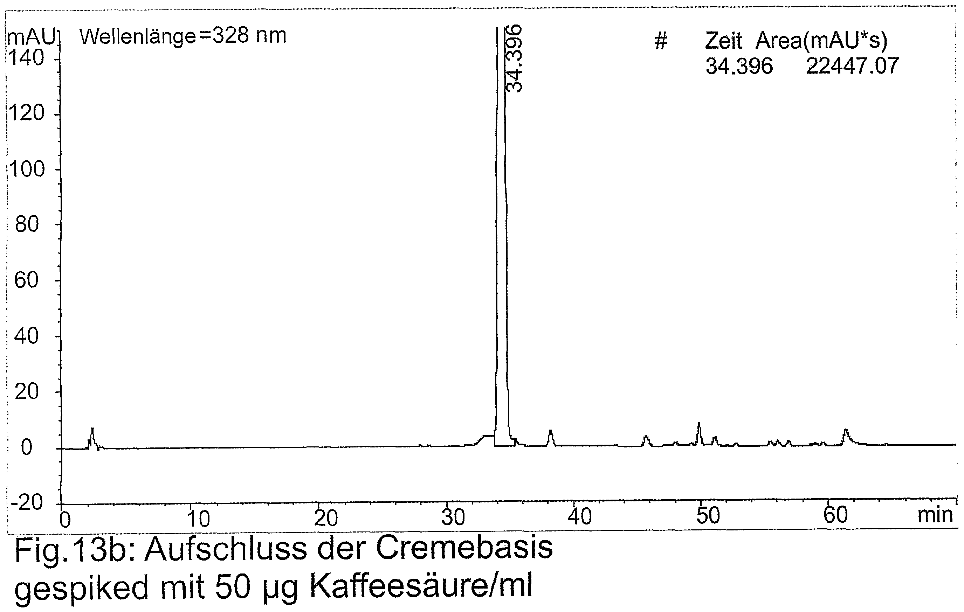

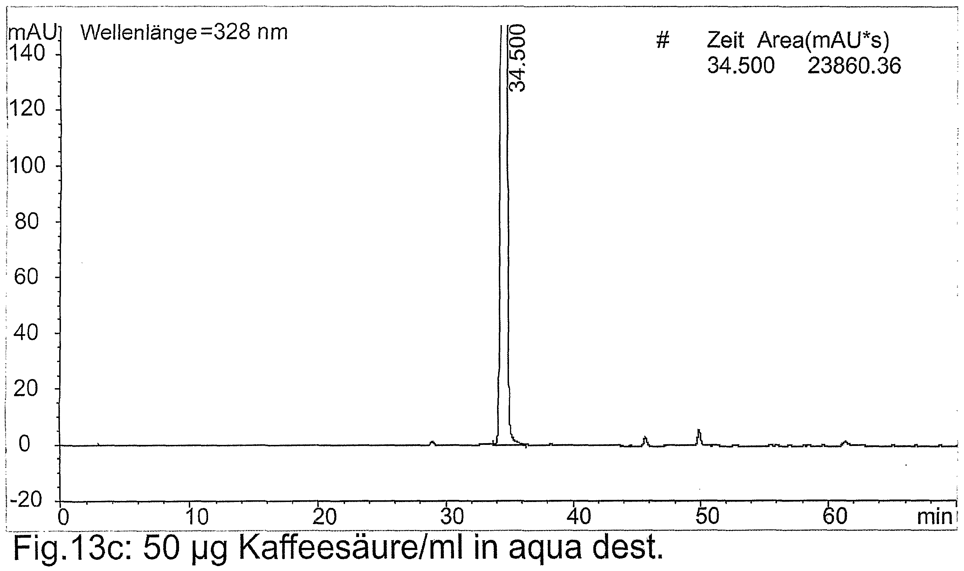

Figs 13a bis c zeigen eine HPLC-Analyse der aufgeschlossenen Creme enthaltend das erfindungsgemäße Extrakt, gespiked mit 50 µg/ml Kaffeesäure (Fig 13a), eine HPLC-Analyse der aufgeschlossenen Cremebasis gespiked mit 50 µg/ml Kaffeesäure (Fig 13b) und eine HPLC-Analyse der Reinsubstanz 50 µg/ml Kaffeesäure (Fig 13c) in Aqua dest.

Aus den Figuren 9 bis 13 und den entsprechenden Kalibrierungsanalysen lassen sich auch die Konzentrationen der verschiedenen Wirkstoffe mittels HPLC in dem Aufschluss der fertigen Creme ermitteln. Nachstehend wird dargestellt, wie aus der integrierten Peakfläche (mAU) mittels Kalibrationskurven die Mengen der Wirksubstanzen berechnet werden:

| | Aufschluss der fertigen Creme in mAU | mAU/1 µg Substanz / ml | µg Substanz/ml | Verhältnis/Faktor |
|---|---|---|---|---|
| Kaffeesäure | 2867,00 | 429,66 | 6,67 | 2,44 |
| p-Coumarinsäure + Cafeoylmalat | 801,00 | 293,24 | 2,73 | 1,00 |
| Coumarin | 347,00 | 116,82 | 2,97 | 1,09 |
| Trans-Ferulasäure | 426,00 | 441,08 | 0,97 | 0,35 |
| Coumarin + trans-Ferulasäure | 773,00 | 278,95 | 3.94 | 1.44 |

Vorzugsweise liegt im Aufschluss der fertigen Creme das Verhältnis von Kaffeesäure : p-Coumarinsäure + Caffeoylmalat : Coumarin + trans-Ferulasäure im Bereich von (1,2 - 3) : (0,8 - 1,2) : (1.0 - 1.8).

### Beispiel 3: Beschreibung der Wirkung und Anwenderbeobachtungen.

Die Kombination der Effekte von Schmerzhemmung, Entzündungshemmung, Durchblutungsförderung und Entkrampfung in dem erfindungsgemäß hergestellten Extrakt hat zu erstaunlich guten Wirkungen geführt.

Anwenderbeobachtungen: Die Anti-Schmerzcreme wurde von mehr als 200 freiwilligen Schmerzpatienten getestet. Die Anwender waren entweder mit ihrer aktuellen Schmerztherapie nicht zufrieden oder hatten schon starke Nebenwirkungen erfahren.

Die Anwendungen und freiwilligen Testpersonen (über 200 ) beziehen sich in erster Linie auf Schmerzen, (z.B. im Gelenksbereich, wie Knie (19), Hüfte (36), Schulter (13), Arthritis (15), Polyarthritis (8), Rückenschmerzen (35), Bandscheibenvorfälle (4), Knochenfrakturen (4), Zahnschmerzen(4), auf Weichteilschmerzen und Sehnen (Zerrungen) (15), Tennisarm, Sehnenentzündungen (9), usw. aber auch Ischias, Hühneraugen, Fersensporn bis hin zu extremen Tumorschmerzen, etc. In allen Fällen kam es sehr rasch zu einer starken Schmerzlinderung bis zu kompletter Schmerzfreiheit schon nach der ersten oder wenigen Anwendungen zumindest für einige Stunden. Erstaunlicherweise konnten einige Testpersonen in kurzer Zeit auch die stärksten Schmerzmittel, wie Duralgesic-Pflaster (Fentanyl = synthetisches Heroin) sehr rasch substituieren.

### Beispiel 3a:

Eine weibliche Testperson (78) hatte vor 25 Jahren einen Sturz aus mehr als 10 Meter Höhe, die dabei erlittene Wirbelfraktur wurde nicht erkannt und daher auch nicht behandelt. Vor 4 Jahren begannen nach 2 leichten Schlaganfällen die Rückenschmerzen immer stärker zu werden und nur fachärztlich verschriebene Duralgesic-Pflaster (synthetisches Heroin) konnten die Schmerzen noch reduzieren. Die Nebenwirkungen hatten allerdings dazu geführt, dass die Frau zusehends immobiler wurde und ihr Bett nicht mehr verlassen konnte. Nach Applikation der erfindungsgemäßen Zubereitung konnte die Testperson innerhalb von drei Tagen wieder alleine aufstehen. Sie hat innerhalb von 14 Tagen die Duralgesic-Pflaster eliminiert und nach 4 Monaten intensiver Anwendung der Creme bedurfte sie auch der Creme nicht mehr und ist seit 1,5 Jahren ohne Anti-Schmerzmittel schmerzfrei.

### Beispiel 3b:

Einer männlichen Testperson (71) wurde 1964 ein ätzendes Kontrastmittel an zwei Stellen in das Rückenmark injiziert um seine Rückenschmerzen zu untersuchen. Unglücklicherweise wurde vergessen das Kontrastmittel, wie üblich, nach der Untersuchung wieder abzusaugen. Die Rückenschmerzen wurden immer intensiver und die Testperson musste über 30 Jahre bis zu 20 Morphiumtabletten pro Tag nehmen, um die Schmerzen einigermaßen erträglich zu halten. In der Nacht musste die Testperson ca. alle 1,5 h eine Morphiumtablette nehmen. Nachdem die Testperson die erfindungsgemäße Creme appliziert hatte, konnte sie wieder 5-7 Stunden schlafen, alleine wieder vom Bett oder einem Stuhl aufstehen und die Dosis an Morphiumtabletten innerhalb von vier Wochen auf 25% reduzieren.

## Patentansprüche

1. Wässriges Brennnesselextrakt umfassend ein Gemisch von p-Coumarinsäure und einem Caffeoylmalat sowie ein Gemisch von Coumarin und trans-Ferulasäure, und zwar mit einer Mindestkonzentration von 0,75 µg/ml an Gemisch von p-Coumarinsäure und einem Caffeoylmalat und einer Mindestkonzentration von 1.00 µg/ml an Gemisch von Coumarin und trans-Ferulasäure, bei Extraktion von 4 g getrocknete und zerkleinerte Blätter und Stängel mit 96 ml RO oder HQ-Wasser, dadurch erhältlich, dass Brennnesseln (Urtica dioica/Urtica urens, Stinging Nettle) mittels Wasser bei einer Temperatur von 120 bis 140°C unter Druck extrahiert werden.

2. Extrakt nach Anspruch 1, **dadurch gekennzeichnet, dass** es unter anderen auch Kaffeesäure umfasst, und zwar in einer Mindestkonzentration von 1,50 µg/ml bei Extraktion von 4 g getrocknete und zerkleinerte Blätter und Stängel mit 96 ml RO oder HQ-Wasser.

3. Extrakt nach Anspruch 1 oder 2, **dadurch gekennzeichnet, dass** es ein Gemisch von p-Coumarinsäure und einem Caffeoylmalat, ein Gemisch von Coumarin und trans-Ferulasäure sowie Kaffeesäure umfasst, und zwar mit einer Mindestkonzentration von 0,90 µg/ml an Gemisch von p-Coumarinsäure und einem Caffeoylmalat, einer Mindestkonzentration von 1,05 µg/ml an Gemisch von Coumarin und trans-Ferulasäure sowie einer Mindestkonzentration von 2,00 µg/ml Kaffeesäure.

4. Extrakt nach einem der Ansprüche 1 bis 3, **dadurch gekennzeichnet, dass** es ein Gemisch von p-Coumarinsäure und einem Caffeoylmalat, ein Gemisch von Coumarin und trans-Ferulasäure sowie Kaffeesäure umfasst, und zwar mit einer Mindestkonzentration von 1,00 µg/ml an Gemisch von p-Coumarinsäure und einem Caffeoylmalat, einer Mindestkonzentration von 1,15 µg/ml an Gemisch von Coumarin und trans-Ferulasäure sowie einer Mindestkonzentration von 2,70 µg/ml Kaffeesäure.

5. Verfahren zur Herstellung des Brennnesselextrakts nach einem der Ansprüche 1 bis 4, **dadurch gekennzeichnet, dass** Brennnesseln (Urtica dioica/Urtica urens, Stinging Nettle) mittels Wasser bei einer Temperatur von 120 bis 140°C unter Druck extrahiert werden.

6. Verfahren nach Anspruch 5, **dadurch gekennzeichnet, dass** zur Extraktion Wasser in einer Menge von 85 bis 99 %-Masse, vorzugsweise 90 bis 97 %-Masse, besonders bevorzugt 96 %-Masse, bezogen auf die Gesamtmasse der zu extrahierenden Zusammensetzung, eingesetzt wird.

7. Verfahren nach Anspruch 5 oder 6, **dadurch gekennzeichnet, dass** die Extraktion über einen Zeitraum von 1 bis 60 min durchgeführt wird.

8. Verfahren nach Anspruch 7, **dadurch gekennzeichnet, dass** die Extraktion über einen Zeitraum von 20 bis 30 min durchgeführt wird.

9. Verfahren nach einem der Ansprüche 5 bis 8, **dadurch gekennzeichnet, dass** 1 bis 10 %-Masse, vorzugsweise 5 bis 7 %-Masse, besonders bevorzugt 4 %-Masse, Brennnesseln (Urtica dioica/Urtica urens, Stinging Nettle, vorzugsweise in Form getrockneter und zerkleinerter Blätter und Stängel, bezogen auf die Gesamtmasse der zu extrahierenden Zusammensetzung, eingesetzt werden.

10. Verwendung des wässrigen Brennnesselextrakts nach einem der Ansprüche 1 bis 4 zur Herstellung einer pharmazeutischen Zubereitung.

11. Verwendung nach Anspruch 10 zur Herstellung einer pharmazeutischen Zubereitung zur Behandlung von Entzündungen, Durchblutungsstörungen und/oder Schmerzen.

## Claims

1. Aqueous stinging nettle extract comprising a mixture of p-Coumaric acid and a caffeoyl malate, and a mixture of coumarin and trans-Ferulic acid, specifically a minimum concentration of 0.75 µg/ml of the mixture of p-Coumaric acid and a caffeoyl malate, and a minimum concentration of 1.00 µg/ml of the mixture of coumarin and trans-Ferulic acid when extracted from 4g of dried and crushed leaves and stems using 96 ml RO or HQ water, which extract can be obtained by extraction from stinging nettles (Urtica dioica/Urtica urens) at a temperature of from 120 to 140°C, under pressure and by means of water.

2. Extract according to claim 1, **characterised in that** it also comprises, *inter alia,* caffeic acid, specifically in a minimum concentration of 1.50 µg/ml when extracted from 4 g of dried and crushed leaves and stems using 96 ml of RO or HQ water.

3. Extract according to either claim 1 or claim 2, **characterised in that** it comprises a mixture of p-Coumaric acid and a caffeoyl malate, a mixture of coumarin and trans-Ferulic acid, as well as caffeic acid, specifically a minimum concentration of 0.90 µg/ml of the mixture of p-Coumaric acid and a caffeoyl malate, a minimum concentration of 1.05 µg/ml of the mixture of coumarin and trans-Ferulic acid, and a minimum concentration of 2.00 µg/ml of caffeic acid.

4. Extract according to any of claims 1 to 3, **characterised in that** it comprises a mixture of p-Coumaric acid and a caffeoyl malate, a mixture of coumarin and trans-Ferulic acid, as well as caffeic acid, specifically in a minimum concentration of 1.00 µg/ml of the mixture of p-Coumaric acid and a caffeoyl malate, a minimum concentration of 1.15 µg/ml of the mixture of coumarin and trans-Ferulic acid, and a minimum concentration of 2.70 µg/ml of caffeic acid.

5. Method for preparing the stinging nettle extract according to any of claims 1 to 4, **characterised in that** stinging nettles (Urtica dioica/Urtica urens) are extracted at a temperature of from 120 to 140°C, under pressure and by means of water.

6. Method according to claim 5, **characterised in that** water in an amount of from 85 to 99 percent by mass, preferably 90 to 97 percent by mass, particularly preferably 96 percent by mass, based on the total weight of the composition to be extracted, is used for the extraction.

7. Method according to either claim 5 or claim 6, **characterised in that** the extraction is carried out over a period of from 1 to 60 minutes.

8. Method according to claim 7, **characterised in that** the extraction is carried out over a period of from 20 to 30 minutes.

9. Method according to any of claims 5 to 8, **characterised in that** stinging nettles (Urtica dioica/Urtica urens), preferably in the form of dried and crushed leaves and stems, are used in an amount of from 1 to 10 percent by mass, preferably 5 to 7 percent by mass, particularly preferably 4 percent by mass, based on the total weight of the composition to be extracted.

10. Use of the aqueous stinging nettle extract according to any of claims 1 to 4 for preparing a pharmaceutical preparation.

11. Use according to claim 10 for preparing a pharmaceutical preparation for treating inflammations, circulation problems and/or pains.

## Revendications

1. Extrait aqueux d'ortie comprenant un mélange d'acide p-coumarique et d'un malate de caféoyle, ainsi qu'un mélange de coumarine et d'acide trans-férulique, et plus précisément présentant une concentration minimale de 0,75 µg/ml du mélange d'acide p-coumarique et d'un malate de caféoyle et une concentration minimale de 1,00 µg/ml du mélange de coumarine et d'acide trans-férulique, lors de l'extraction de 4 g de feuilles et de tiges séchées et broyées avec 96 ml d'eau Ol ou HQ, pouvant être obtenu par extraction d'orties (Urtica dioica/Urtica urens, stinging nettle) à l'aide d'eau à une température de 120 à 140°C sous pression.

2. Extrait selon la revendication 1, **caractérisé en ce qu'**il comprend entre autres aussi de l'acide caféique, et plus précisément à une concentration minimale de 1,50 µg/ml, lors de l'extraction de 4 g de feuilles et de tiges séchées et broyées avec 96 ml d'eau Ol ou HQ.

3. Extrait selon la revendication 1 ou 2, **caractérisé en ce qu'**il comprend un mélange d'acide p-coumarique et d'un malate de caféoyle, un mélange de coumarine et d'acide trans-férulique, ainsi que de l'acide caféique, et plus précisément présentant une concentration minimale de 0,90 µg/ml du mélange d'acide p-coumarique et d'un malate de caféoyle, une concentration minimale de 1,05 µg/ml du mélange de coumarine et d'acide trans-férulique, et une concentration minimale de 2,00 µg/ml d'acide caféique.

4. Extrait selon l'une des revendications 1 à 3, **caractérisé en ce qu'**il comprend un mélange d'acide p-coumarique et d'un malate de caféoyle, un mélange de coumarine et d'acide trans-férulique, ainsi que de l'acide caféique, et plus précisément présentant une concentration minimale de 1,00 µg/ml du mélange d'acide p-coumarique et d'un malate de caféoyle, une concentration minimale de 1,15 µg/ml du mélange de coumarine et d'acide trans-férulique, ainsi qu'une concentration minimale de 2,70 µg/ml d'acide caféique.

5. Procédé de fabrication d'un extrait d'ortie selon l'une des revendications 1 à 4, **caractérisé en ce qu'**on extrait des orties (Urtica dioica/Urtica urens, stinging nettle) à l'aide d'eau à une température de 120 à 140°C sous pression.

6. Procédé selon la revendication 5, **caractérisé en ce qu'**on utilise pour l'extraction de l'eau en une quantité de 85 à 99 % en masse, de préférence de 90 à 97 % en masse, d'une manière particulièrement préférée de 96 % en masse, par rapport à la masse totale de la composition à extraire.

7. Procédé selon la revendication 5 ou 6, **caractérisé en ce que** l'extraction est mise en oeuvre sur une durée de 1 à 60 min.

8. Procédé selon la revendication 7, **caractérisé en ce que** l'extraction est mise en oeuvre sur une durée de 20 à 30 min.

9. Procédé selon l'une des revendications 5 à 8, **caractérisé en ce qu'**on utilise 1 à 10 % en masse, de préférence 5 à 7 % en masse, d'une manière particulièrement préférée 4 % en masse d'orties (Urtica dioica/Urtica urens, stinging nettle), de préférence sous forme de feuilles et de tiges séchées et broyées, par rapport à la masse totale de la composition à extraire.

10. Utilisation de l'extrait aqueux d'ortie selon l'une des revendications 1 à 4 pour la fabrication d'une préparation pharmaceutique.

11. Utilisation selon la revendication 10 pour la fabrication d'une préparation pharmaceutique destinée au traitement d'inflammations, de troubles de la circulation sanguine et/ou de douleurs.
